# EUROPEAN PATENT APPLICATION

(11) **EP 3 251 711 A1**
(43) Date of publication of application: **06.12.2017**
(21) Application number: 16305633.6
(22) Date of filing: 01.06.2016
(51) Int. Cl.: A61M 5/168

(54) **ALARM SYSTEM OF A MEDICAL DEVICE AND METHOD FOR OPERATING AN ALARM SYSTEM**

(71) Applicant: Fresenius Vial SAS, 38590 Brézins (FR)
(72) Inventor: VALLOIS, Clément, 38500 Saint Cassien (FR)
(74) Representative: Kusche, Robert

(57) **Abstract**

An alarm system (15) of a medical device (1) comprises a processor device (150) constituted to determine whether a predefined alarm condition is present, a signalling device (153) for outputting an alarm signal (S) in case the predefined alarm condition is present, and a storage device (151). Herein, the processor device (150) is constituted to determine a response time (T) between a first time (t1) associated with the beginning of the outputting of the alarm signal (S) and a second time (t2) associated with a response of a user to the alarm condition, wherein the storage device (151) is constituted to store the response time (T). In this way an alarm system of a medical device is provided which allows to improve the user's awareness to the occurrence of an alarm condition.

## Description

The invention relates to an alarm system of a medical device according to the preamble of claim 1 and to a method for operating an alarm system.

An alarm system of this kind comprises a processor device constituted to determine whether a predefined alarm condition is present. A signaling device outputs an alarm signal in case the predefined alarm condition is present. Parameters for operating the alarm system may be stored in a storage device, for example parameters relating to one or multiple predefined alarm conditions.

A medical device of this kind may for example be constituted as an infusion device, for example a syringe pump or a volumetric (peristaltic) infusion pump. With a medical device of this kind a medical fluid such as a medication, a nutritional fluid for the enteral or parenteral feeding of a patient or another medical solution such as a saline solution can be administered to a patient.

When operating a medical device such as an infusion device, for example unsatisfactory physiological patient states should be detected in order to, possibly, initiate suitable countermeasures. For example, if during the infusion of a medical fluid to a patient an occlusion of an infusion line occurs, an alarm condition must be raised warning a user of the occlusion such that the user, for example a nurse, may react to the alarm and may take action to release the alarm condition, for example by releasing the occlusion.

Multiple different alarm conditions herein may be defined and may occur, for example relating to an empty bag condition, a disruption or malfunction in the power supply or an abnormality of an operational parameter of for example an infusion device during the programming of the infusion device or during the actual infusion operation.

An alarm condition may for example be present if an operational parameter exceeds a threshold. For example, an occlusion in an infusion line may be detected by monitoring a pressure in the infusion line, wherein an alarm condition may be raised if the pressure in the infusion line exceeds a predetermined threshold. In case an alarm condition is present, an alarm signal may be issued, for example by sounding an acoustic alarm and, in addition or alternatively, by outputting a visual alarm for example by blinking a light or by outputting an indication on a display of a medical device.

At a bed side of a patient, for example in an intensive care unit of a hospital, a multiplicity of medical devices such as infusion devices may be placed. Hence, multiple medical devices may output different alarm signals at the same time, such that a user, for example a nurse, may be faced with multiple alarm conditions indicated by multiple alarm signals at the same time. Alarm conditions herein may have different priorities and correspondingly may be associated with different alarm signals chosen according to their different priorities. The occurrence of an alarm signal may, to a user, actually be rather common in particular for alarm conditions of lower priorities, such that a user may be used to the occurrence of an alarm condition and may hence react to the alarm condition with a reduced awareness. This may lead to certain alarm conditions to be not attended to at all, such that alarm conditions may be missed.

It is an object of the instant invention to provide an alarm system of a medical device and a method for operating an alarm system which allow to improve the user's awareness to the occurrence of an alarm condition.

The object is achieved by means of an alarm system comprising the features of claim 1.

Accordingly, the processor device of the alarm system is constituted to determine a response time between a first time associated with the beginning of the outputting of the alarm signal and a second time associated with a response of a user to the alarm condition, wherein the storage device is constituted to store the response time or a value derived from the response time.

The processor device hence determines a response time of a user to respond to an alarm condition. The processor device hence determines the time duration the user needs to react to the alarm condition and stores this duration or a value derived from the measured duration, for example an average response time, in a storage device.

If the response time of the user is known, it may be used for various purposes. For example, from the response time it may be analyzed how long it takes a user on average to react to a specific alarm condition. Hence, it may be evaluated for different alarm conditions how long a user needs to on average respond to the alarm condition. Such evaluation data may be read out from the storage device, for which a user may access the medical device locally via an input/output device of the medical device or remotely by accessing the medical device for example via a communication network to which the medical device is connected. According to the evaluation data it then is possible to for example reorganize a workflow in a hospital, for example within a ward of a hospital, such that it is taken care that staff of the hospital may suitably respond to alarm conditions of medical devices.

The response time may also be used by the alarm system of the medical device itself to automatically adapt the operation of the alarm system. For example, the processor device may be constituted to adapt the alarm signal associated with the alarm condition in dependence of the response time or an average response time determined from a multiplicity of response times. For example, if it is detected that a response time or an average response time determined from a multiplicity of response times exceeds a predefined threshold, the alarm signal associated with the alarm condition may be adapted for example by changing the tone or frequency of an acoustic alarm signal or by increasing the volume of an acoustic alarm signal. If it hence is detected that it takes a user too long to react to a certain alarm signal associated with an particular alarm condition, the alarm signal may be changed in a way that that the user's awareness to the alarm signal is improved. In addition or alternatively, the kind of alarm signal may be changed. If for example previously only an acoustic alarm signal was issued, the alarm signal may be adapted such that an additional alarm signal such as a visual alarm signal is output or an additional alarm message is sent to a user via a communication network.

Alarm conditions generally may be assigned different priorities. For example, different levels of alarm priorities may exist relating to for example system advisories (having a low priority), caution alarms (having a mid-level priority) and warning alarms (having a high priority). In dependence of the priorities of the alarm conditions, different alarm signals may be issued, the alarm signals transporting a different sense of urgency for alerting a user. For example, a low level alarm condition may cause only a visual alarm signal output for example via a display of a medical device and stating the alarm condition for example by text on the display. A mid-level priority may be associated with a low volume acoustic alarm signal. And a high level priority alarm condition may be associated with an alarm signal comprising an acoustic alarm of a high volume as well as a visual alarm such as for example a blinking light or the like.

The processor device may be constituted, in one embodiment, to change the priority associated with an alarm condition in dependence of the response time or an average response time determined from a multiplicity of response times. For example, if it is found that the response time or the average response time exceeds a predefined threshold, the priority associated with the alarm condition may be increased, and correspondingly an alarm signal associated with the increased priority may be output to increase the awareness of the user to the alarm condition.

The alarm system may comprise, in one embodiment, an actuation device actuatable by a user for responding to the alarm condition. The actuation device may for example have the shape of a button which a user may press in response to an alarm condition. The actuation device may for example cause an alarm signal to be silenced, for example by switching the alarm signal off or by muting the alarm signal for a predetermined time period after which the alarm signal reoccurs.

The time until which the response time is measured may also correspond to the moment at which a user releases the alarm condition, for example by releasing an occlusion in an infusion line or by altering an operational parameter of the medical device. If the alarm condition is no longer present, the alarm signal may be switched off and may reoccur only if the alarm condition occurs again.

The time from which the response time is measured may for example correspond to a time at which the signaling device begins to output the alarm signal. Herein, it is also possible to include a predefined delay such that the response time is measured from a moment in time a predefined delay after the beginning of the outputting of the alarm signal.

The processor device may be constituted, in one aspect, to determine an average response time from a multiplicity of response times associated with a particular alarm condition or associated with a multiplicity of different alarm conditions. For example, an average response time may be determined from a predefined number of response times associated with a particular alarm condition such that an average response time for the particular alarm condition is obtained. In addition or alternatively, an average response time from a multiplicity of response times of alarm conditions associated with a particular priority may be determined such that an average response time for all alarm conditions of a particular priority is obtained. In addition or alternatively, an average response time of all alarm conditions may be determined such that an average response time for all different alarm conditions defined in the medical device is obtained.

The alarm system may be part of a medical device, for example an infusion device. The alarm system may be implemented for example by software such that the alarm system may use for example a processor device of the medical device and a storage device of the medical device. It however is also conceivable that the alarm system is separate from a medical device and is implemented for example by a standalone device having its own processor device and storage device.

The object is also achieved by a method for operating an alarm system of a medical device, comprising:
- determining, using a processor device, whether a predefined alarm condition is present, and
- outputting, using a signalling device, an alarm signal in case the predefined alarm condition is present.

Herein, a response time between a first time associated with the beginning of the outputting of the alarm signal and a second time associated with a response of a user to the alarm condition is determined using the processor device, wherein the response time or a value derived from the response time is stored in a storage device.

The advantages and advantageous embodiments described above for the alarm system equally apply also to the method such that it shall be referred to the above.

The idea of the invention shall subsequently be described in more detail with reference to the embodiments shown in the figures. Herein:
- Fig. 1: shows a view of an infusion device constituted as a syringe pump;
- Fig. 2: a flow chart illustrating the processing of an alarm condition; and
- Fig. 3: a diagram indicating a response time between the beginning of the outputting of an alarm signal and a user's response to the alarm signal.

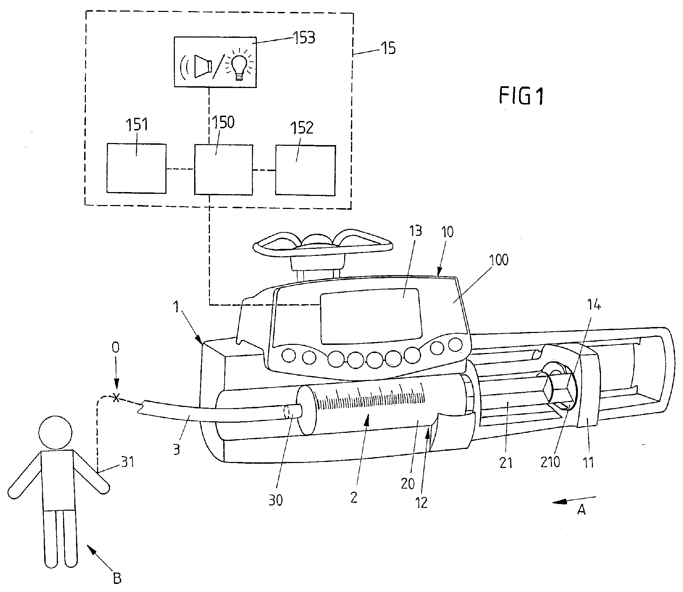

Fig. 1 shows an embodiment of a medical device constituted as an infusion device 1 in the shape of a syringe pump. The infusion device 1 comprises a housing 10 having a front face 100 and a display device 13 arranged thereon. The display device 13 may for example be a touch-sensitive display allowing a user to enter commands for operation of the infusion device 1 and displaying operational information regarding the process of an actual infusion operation.

The infusion device 1 comprises a receptacle 12 in which a syringe 2 having a cylindrical tube 20 is arranged. A piston 21 is movable within the cylindrical tube 20 and is in engagement with a pusher device 11 of a pumping mechanism of the infusion device 1. At an end of the cylindrical tube 20 opposite the piston 21 a delivery line 3 extends from the cylindrical tube 20 towards a patient B, the delivery line 3 being connected to the cylindrical tube 20 at an end 30 and to the patient B at an end 31.

The piston 21 comprises a head 210 facing away from the cylindrical tube 20 and being in abutment with the pusher device 11 of the infusion device 1. During operation of the infusion device 1, the pusher device 11 is electromotorically driven in an actuation direction A such that the piston 21 is moved into the cylindrical tube 20 and a medical fluid contained in the cylindrical tube 20 is delivered via the delivery line 3 towards the patient B.

During an infusion process a medical fluid, for example a medication or a nutritional fluid for the parenteral feeding of a patient or the like, is delivered from the cylindrical tube 20 via the delivery line 3 towards the patient B. For this, the piston 21 is continuously pushed into the cylindrical tube 20 in the actuation direction A such that a desired flow rate is obtained, which is programmed by a user prior to the start of the infusion operation.

The delivery line 3 generally is made of a flexible tubing made for example from a PVC material. The delivery line extends from the cylindrical tube 20 to the patient B and is, at its first end 30, in fluid connection with the cylindrical tube 20 and, at its second end 31, for example connected to a needle for providing an intravenous access to the patient B. During an infusion process an occlusion O in the delivery line 3 must be avoided and, if it nevertheless occurs, must be detected such that appropriate countermeasures to overcome the occlusion O can be taken. For this, a sensor device in the shape of a force sensor 14 is placed on the pusher device 11 facing the head 210 of the piston 214 measuring a force exerted on the piston 21 during an infusion process. From a force measured by means of the force sensor 14 an estimate of the pressure within the delivery line 3 connected to the syringe 2 can be obtained, such that the pressure within the delivery line 3 can be monitored. If it is found that the pressure within the delivery line 3 rises beyond a permissible threshold value, an alarm is triggered indicating that an occlusion O may be present in the system.

The infusion device 1 comprises or is linked to an alarm system 15 comprising a processor device 150, a storage device 151, an actuation device 152 actuatable by a user and a signaling device 153 for outputting an alarm signal. The alarm system 15 may be integrated into the infusion device 1 and may be implemented for example by software, making use for example of a processor device 150 which also controls the operation of the infusion device 1. Alternatively, the alarm system 15 may be implemented by a stand-alone device separate to the infusion device 1 and may be linked to the infusion device 1 for example via a communication network.

The alarm system 15 serves to monitor the infusion device 1 for alarm conditions and to trigger an alarm signal if an alarm condition is detected. The alarm system 15 herein may be constituted to monitor different kinds of alarm conditions, for example during the initial programming of the infusion device 1 prior to performing an infusion operation and during the actual operation of the infusion device 1 for administering a medical fluid to a patient B.

Different alarm conditions herein may have different priorities. For example, advisory alarms may advise a user for example during the programming of the infusion device 1 that a value of an operational parameter, for example the infusion rate, is out-of-bounds and should be chosen differently. Such advisory alarms may have a low priority. A caution alarm may have a mid-level priority and may be triggered during the programming of the infusion device 1 or during operation of the infusion device 1 advising a user that caution should be applied when proceeding for example with an infusion operation. And a warning alarm may have a high priority for indicating for example a malfunction during operation of the infusion device 1, for example due to an occlusion O present in an infusion line 3 or for example due to an instability in the power supply or the like.

Generally, the processor device 150 determines whether a predefined alarm condition is present. For example, the processor device 150 compares a pressure in the infusion line 3 derived from sensor readings of the force sensor 14 with a predefined pressure threshold and, if the pressure threshold is exceeded, concludes that an occlusion O is present in the infusion line 3. Consequently, an alarm condition is present, and the signaling device 153 is triggered to output an alarm signal, for example an acoustic alarm signal and/or a visual alarm signal.

Once an alarm condition is present and an alarm signal is output by the signaling device 153, a user should react to the alarm signal, preferably by releasing the alarm condition, for example by releasing the occlusion O in the infusion line 3. In addition or alternatively a user may actuate the actuation device 152, which for example may comprise a button which the user may press to silence the alarm signal permanently or to mute the alarm signal for a predetermined time period after which the alarm signal reoccurs.

Generally, multiple medical devices such as multiple infusion devices 1 may be placed at the bedside of a patient B for example in a hospital environment, for example in an intensive care unit of a hospital. Different medical devices herein may experience different alarm conditions at the same time, such that a user may be simultaneously faced with different alarm conditions, each associated with an acoustic and/or visual alarm.

The occurrence of different alarm conditions at the same time as well as the repeated reoccurrence of alarm conditions may cause a user to only slowly respond to an alarm condition. Just as well, there is a risk that an alarm condition is missed and not attended to at all by a user, because other alarm conditions may have a higher priority and may supersede a low priority alarm condition. There hence is a general desire to improve a user's response to alarm conditions occurring on one or multiple medical devices.

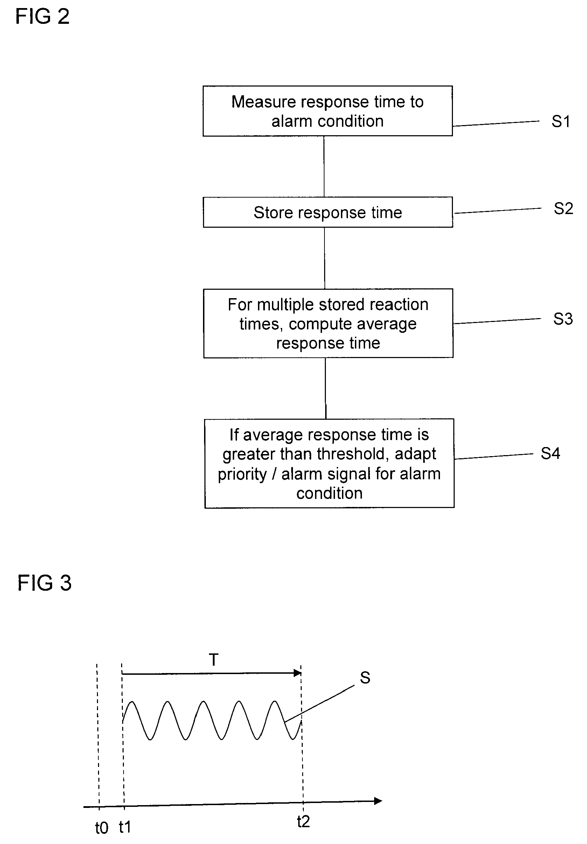

To address this issue, it is proposed to automatically measure and analyze a response time of a user to an alarm condition. In particular, the processor device 150 may be constituted to determine, as illustrated in Fig. 3, a response time T between a first time t1 associated with the beginning of the outputting of an alarm signal S and a second time t2 associated with a response of a user to the alarm condition. Herein, it is assumed that an alarm condition occurs at time t0. Shortly after the occurrence of the alarm condition at time t0, the signaling device 153 is triggered by the processor device 150 to output the alarm signal S associated with the alarm condition, the outputting of the alarm signal S starting at time t1. The processor device 150 automatically determines the time duration of the response time T between the time t1 at which the alarm signal S begins and the time t2 at which a user responds to the alarm signal S, for example by actuating the actuation device 152 or by releasing the alarm condition, for example by releasing an occlusion O in the infusion line 3.

As illustrated in the flow chart of Fig. 2, the processor device 150 in step S1 determines the response time T to an alarm condition. The response time T in step S2 is stored in the storage device 151. For multiple response times T, then, in step S3 an average response time is computed. And if in step S4 it is determined that the average response time is greater than a predefined threshold, the alarm signal S associated with the alarm condition may be adapted or the priority of the alarm condition may be increased, also leading to a different alarm signal S.

Generally, in step S3 the average response time for responding to a particular alarm condition may be computed. If it is determined, that this average response time exceeds the threshold in step S4, the alarm signal and/or the priority for the particular alarm condition may be adapted.

In addition or alternatively, in step S3 in an average response time may also be determined for different alarm conditions associated with the same priority. If it is found for this average response time relating to the alarm conditions of the same priority that a threshold is exceeded, the alarm signals for the alarm conditions may be adapted, for example by increasing the volume of all acoustic alarm signals associated with the alarm conditions.

In addition or alternatively, in step S3 the average response time of all alarm conditions defined for the medical device may be computed. If it is found that the average response time exceeds a threshold, for example the volume of all acoustic alarm signals associated with the different alarm conditions may be increased.

The response time T and the different average response times may also be used for analysis and may be accessed by a user locally via the medical device or remotely via a communication network to which the medical device is connected. The data stored in the storage device 151 in this way may be used to obtain a feedback about the organization within a hospital, for example within a ward of a hospital, such that measures to improve the organization may be initiated if it is found that generally the response to alarm conditions of medical devices in a hospital ward is too slow.

Generally, if it is found that for example an average response time, for example taken over 5, 10 or 20 response times T associated with a particular alarm condition, exceeds a predefined threshold, an acoustic alarm signal may be adapted in its tone, frequency or volume. For example, if an acoustic alarm signal comprises a multiplicity of distinct beeps, the repeat rate of the beeps as well as their tone and volume may be adapted to adjust the sound of the alarm signal. In addition or alternatively, an additional alarm signal such as a visual alarm signal may be triggered together with the acoustic alarm signal.

It also is conceivable that the priority of an alarm condition is adjusted. If for example an alarm condition is associated with a low priority, but if it is found that the response time T to that alarm condition generally is too long, the priority for the alarm condition may be increased for example to a mid-level priority, such that a higher level priority alarm signal S is output upon occurrence of the alarm condition.

The idea underlying the invention is not limited to the embodiments described above, but may be implemented in an entirely different fashion.

The adaption of the alarm signal or the priority of the alarm condition may take place during the output of the alarm signal upon occurrence of an alarm condition, or after analysis of response times in response to a multiplicity of occurrences of alarm conditions. The adaption of the alarm signal or the priority associated with an alarm condition herein may take place automatically by the processor device, without a need for a user interaction.

The number of response times to be averaged (for example 5, 10 or 20) may for example be set by a user. Also, the threshold for adapting the alarm signal and/or the priority of an alarm condition may be user configurable.

An alarm system as described herein may be used on medical devices of different kinds and in particular is not limited to infusion devices as described according to one embodiment above.

### List of Reference Numerals

- 1: Infusion device
- 10: Housing
- 100: Front face
- 11: Pusher device
- 12: Receptacle
- 13: Display device
- 14: Force sensor
- 15: Alarm system
- 150: Processor device
- 151: Storage device
- 152: Actuation device
- 153: Signalling device
- 2: Pumping device (syringe)
- 20: Cylindrical tube
- 21: Piston
- 210: Piston head
- 3: Delivery line
- 30,31: End
- A: Actuation direction
- B: Patient
- O: Occlusion
- S1-S4: Steps
- t0-t2: Time point
- T: Response time

## Claims

1. Alarm system (15) of a medical device (1), comprising:
- a processor device (150) constituted to determine whether a predefined alarm condition is present,
- a signalling device (153) for outputting an alarm signal (S) in case the predefined alarm condition is present, and
- a storage device (151),
**characterized in**
**that** the processor device (150) is constituted to determine a response time (T) between a first time (t1) associated with the beginning of the outputting of the alarm signal (S) and a second time (t2) associated with a response of a user to the alarm condition, wherein the storage device (151) is constituted to store the response time (T) or a value derived from the response time (T).

2. Alarm system (15) according to claim 1, **characterized by** an actuation device (152) actuatable by a user for responding to the alarm condition, wherein the second time (t2) corresponds to a point in time at which the user actuates the actuation device (152).

3. Alarm system (15) according to claim 1 or 2, **characterized in that** the first time (t1) corresponds to a time at which the signalling device (153) begins to output the alarm signal (S).

4. Alarm system (15) according to one of claims 1 to 3, **characterized in that** the processor device (150) is constituted to adapt the alarm signal (S) associated with the alarm condition in dependence of the response time (T) or an average response time determined from a multiplicity of response times (T).

5. Alarm system (15) according to claim 4, **characterized in that** the processor device (150) is constituted to adapt the alarm signal (S) associated with the alarm condition if the response time (T) or an average response time determined from a multiplicity of response times (T) exceeds a predefined threshold.

6. Alarm system (15) according to claim 4 or 5, **characterized in that** the alarm signal (S) is an acoustic alarm signal, wherein the processor device (150) is constituted to adapt the tone, volume or frequency of the alarm signal (S).

7. Alarm system (15) according to one of the preceding claims, **characterized in that** the predefined alarm condition is assigned a predetermined priority out of a multiplicity of predetermined priorities, wherein the processor device (150) is constituted to assign the alarm condition to a different priority in dependence of the response time (T) or an average response time determined from a multiplicity of response times (T).

8. Alarm system (15) according to claim 7, **characterized in that** the processor device (150) is constituted to assign the alarm condition to a different priority if the response time (T) or an average response time determined from a multiplicity of response times (T) exceeds a predefined threshold.

9. Alarm system (15) according to one of the preceding claims, **characterized in that** the processor device (150) is constituted to determine an average response time from a multiplicity of response times (T) associated with a particular alarm condition or associated with a multiplicity of different alarm conditions.

10. Method for operating an alarm system (15) of a medical device (1), comprising:
- determining, using a processor device (150), whether a predefined alarm condition is present, and
- outputting, using a signalling device (153), an alarm signal (S) in case the predefined alarm condition is present,
**characterized in**
**that** a response time (T) between a first time (t1) associated with the beginning of the outputting of the alarm signal (S) and a second time (t2) associated with a response of a user to the alarm condition is determined using the processor device (150), wherein the response time (T) or a value derived from the response time (T) is stored in a storage device (151).
